# EUROPEAN PATENT SPECIFICATION

(11) **EP 2 991 766 B1**
(45) Date of publication and mention of the grant of the patent: **17.01.2018**
(21) Application number: 14791838.7
(22) Date of filing: 25.04.2014
(51) Int. Cl.: B01L 3/00, A61B 10/00

(54) **BODILY FLUID SPECIMEN COLLECTION AND ASSAY DEVICE**
KÖRPERFLÜSSIGKEITSPROBENAHME UND TESTVORRICHTUNG
DISPOSITIF DE COLLECTE ET D'ANALYSE D'ÉCHANTILLON DE FLUIDE CORPOREL

(30) Priority: 30.04.2013 US 201361817414 P
(43) Date of publication of application: 09.03.2016
(73) Proprietor: Microtri Limited, Hong Kong 999077 (CN)
(72) Inventor: SCHWYN, Bernhard, Andreas, Banglamung Chonburi 20250 (TH); TYLER, Glenn, Norman, Banglamung Chonburi 20250 (TH)
(74) Representative: Patentanwaltskanzlei Matschnig & Forsthuber OG
(86) International application number: PCT/IB2014/000755
(87) International publication number: WO 2014/177927

(56) References cited:
- WO-A1-2010/003310
- CN-A- 1 879 016
- CN-A- 102 087 295
- CN-A- 102 680 276
- US-A1- 2004 133 128
- US-A1- 2007 259 442
- US-A1- 2010 278 692

## Description

### Technical Field

This invention relates to a bodily fluid specimen collection and assay device.

### Background of the Invention

The practice of testing bodily fluid specimens for health abnormalities and or evidence of the consumption of a number of illicit and illegal drugs has seen the development of a variety of specimen collection and assay devices for use at home, in the workplace, hospitals, law enforcement authorities, sporting arenas and more recently at social gathering places. These devices can be used for a number of purposes including the detection of illicit drugs such as cocaine, heroin, amphetamine, methamphetamines, morphine, marijuana, opiates, THC, AMP, PCP, sporting performance enhancement drugs and hormones. Many of the testing devices for these drugs are complex in design and difficult to use. They are manufactured using multiple grade raw materials creating disposal and recycling difficulties. Furthermore, they are expensive to produce and do not offer total tamper proof security.

PCT/US2004/038428 disclosed a sample collection cup with integrated sample analysis system where the chamber with strips for testing is contained in the lid. The lid is separate from the sample collection cup. When depositing the bodily fluid into the cup, the user must find a place to put the lid. This is a burden for the user and potentially cause sample contamination as the lid may become contaminated if placed in an unclean area.

US 8,394,626 B2 disclosed a specimen collection and assay container with a separate lid. The lid is a tamper resistant seal with a series of sloped projections. A separated lid is cumbersome for the user and the series of sloped projections do not visually show if the lid has been tampered with. A more practical specimen collection and assay device with better tampering prevention or better tampered-evident is desirable.

Also document CN102680276 relates to a bodily fluid specimen collection and assay device, comprising a sample collection chamber, a transparent sidewall, a test board and a lid which may screwed to a bodily fluid collection cup.

### Summary of the Invention

The present invention is defined by the device of independent claim 1 and is directed to a bodily fluid specimen collection and assay device having a bodily fluid collection cup comprising a bottom and a surrounding wall defining a main collection chamber and an assaying test strip viewing window define on the said wall of the bodily fluid collection cup comprising a damming rib. The bodily fluid specimen collection and assay device has an integral hinged lid integrally hinged to the said bodily fluid collection cup, a magazine supporting member disposed within the main collection chamber. The said magazine supporting member comprises a plurality of flow ports disposed at the lower section of the magazine supporting member. The bodily fluid specimen collection and assay device has a test strip magazine comprising: a plurality of recessed slots each of which is configured to accommodate an assaying test strip, a damming device disposed on each recessed slot, an outward flow port disposed on each damming device; disposed at a position higher than the position of the flow ports on the magazine supporting member; and a raised rib positioned to match the height of the damming rib. The damming rib, the raised rib, the test strip magazine and the magazine supporting member are engaged to create a differential pressure chamber.

Preferred embodiments are defined by the dependent claims.

In an embodiment, the bodily fluid specimen collection and assay device and the integral hinged lid are prepared with corresponding coupling element configured to provide a leak proof permanent seal to the bodily fluid specimen collection and assay device.

In another embodiment, the bodily fluid specimen collection and assay device has an extraction port prepared on the integral hinged lid.

In yet another embodiment, the bodily fluid specimen collection and assay device comprises a drainage port prepared on the bottom of the main collection chamber of the bodily fluid collection cup.

In an embodiment, the bodily fluid specimen collection and assay device comprises an extraction port prepared on the integral hinged lid and a drainage port prepared on the bottom of the main collection chamber of the bodily fluid collection cup.

In yet another embodiment, the bodily fluid specimen collection and assay device comprises an integral hinged lid with a protruding rib support along top of the test strip magazine.

In an embodiment, the bodily fluid specimen collection and assay device comprises an integral hinged lid with a protruding rib configured to rest on the protruding rib support on the integral hinged lid as the integral hinged lid is coupled to the bodily fluid collection cup.

In another embodiment, the bodily fluid specimen collection and assay device comprises recessed slot of a shape corresponding to a shape of a test strip.

In an embodiment, the bodily fluid specimen collection and assay device comprises test strip magazine with up to eight recessed slots.

In another embodiment, the bodily fluid specimen collection and assay device comprises at least an area for identification labeling.

In an embodiment, the- bodily fluid specimen collection and assay device - comprises an extraction port comprising a self-resealed polymeric material or membrane.

### Brief Description of the Drawings

Advantages and characteristics of the invention will be appreciated from the following description, in which, as a non-limiting example, some preferable embodiments of the principle of the invention are described, with reference to the accompanying drawings, in which:
Fig. 1 illustrates a perspective view of an embodiment of the bodily fluid specimen collection and assay device showing the hinged lid open;
Fig. 2 illustrates a sectional perspective view of an embodiment of the bodily fluid specimen collection and assay device showing the hinged lid open; and a drainage port;
Fig. 3 illustrates an embodiment of the bodily fluid specimen collection and assay device hinged lid closed; and an extraction port defined on the hinged lid;
Fig. 4 illustrates a scrap sectional view of the embodiment of the bodily fluid specimen collection and assay device of FIG. 3 and its corresponding coupling element with coupling profiles between the bodily fluid collection cup and the integral hinged lid;
Fig. 5 illustrates an embodiment of front of a test strip magazine;
Fig. 6 illustrates an embodiment of back of a test strip magazine;
Fig. 7 illustrates a sectional perspective view of an embodiment of the bodily fluid specimen collection and assay device with the hinged lid closed and showing the outward flow port of the damming device in relation to the flow port of the magazine supporting member;
Fig. 8 illustrates a scrap sectional view of the embodiment of FIG. 7 illustrating direction of flow of the bodily fluid specimen through the test strip magazine;
Fig. 9 illustrates a scrap sectional view of the embodiment of FIG. 7 illustrating sample filled bodily fluid specimen collection and assay device and flow of the bodily fluid specimen through the test strip magazine; and
Fig. 10 illustrates an embodiment of the bodily fluid specimen collection and assay device illustrating an assaying test strip viewing window with assaying test strips placed within the bodily fluid specimen collection and assaying device, assaying test strip conjugate zone and reaction zone.

### Detailed Description of Preferred Embodiment of the Invention

The present invention is directed to a bodily fluid specimen collection and assay device having a bodily fluid collection cup comprising a bottom and a surrounding wall defining a main collection chamber and an assaying test strip viewing window define on the said wall of the bodily fluid collection cup comprising a damming rib. The bodily fluid specimen collection and assay device has an integral hinged lid integrally hinged to the said bodily fluid collection cup, a magazine supporting member disposed within the main collection chamber. The said magazine supporting member comprises a plurality of flow ports disposed at the lower section of magazine supporting member. The bodily fluid specimen collection and assay device has a test strip magazine comprising: a plurality of recessed slots each of which is configured to accommodate an assaying test strip, a damming device disposed on each recessed slot, an outward flow port disposed on each damming device; disposed at a position higher than the position of the flow ports on the magazine supporting member; and a raised rib positioned to match the height of the damming rib. The damming rib, the raised rib, the test strip magazine and the magazine supporting member are engaged to create differential pressure chamber.

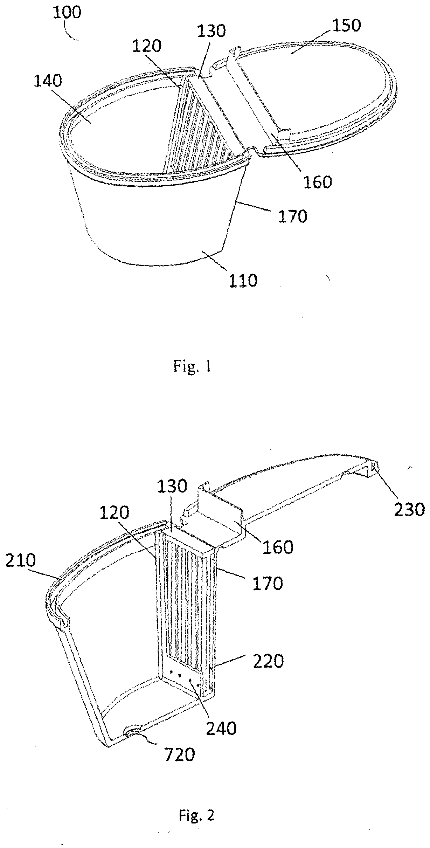

Figs. 1- 10 show a bodily fluid specimen collection and assay device **100** according to the principle of the invention.

In more detail, Fig. 1 illustrates an embodiment of the bodily fluid specimen collection and assay device **100.** The bodily fluid specimen collection and assay device **100** will now be referred to as "device." The device **100** comprises a bodily fluid collection cup **110** and an integral hinged lid **150** integrally hinged to the bodily fluid collection cup **110.** The bodily fluid collection cup **110** will now be referred to as the "collection cup." The collection cup **110** comprises a bottom wall with a surrounding wall to define a main collection chamber **140.** The said wall defines an assaying test strip viewing window **170** to facilitate viewing of the test results which will be further described below. The collection cup **110** also comprises a magazine supporting member **120** disposed within the main collection chamber **140** at a corresponding position at which the assaying test strip viewing window **170** is located, such that a pocket between the wall of the collection cup **110** which is designated as the assaying test strip viewing window **170** and the magazine supporting member **120** is formed so as to accommodate a test strip magazine **130** to be inserted therein. Both collection cup **110** and the integral hinged lid **150** are prepared with a corresponding coupling element such that a leak proof permanent seal is achieved once the integral hinged lid is engaged with the collection cup **110.** Further, the integral hinged lid **150** is not only configured to securely couple with the collection cup **110** so as to contain a sample, but also functioning as a handle during the act of depositing a bodily fluid specimen such as urine into the collection cup **110.** The integral hinged lid **150** retains its post-molded rigidity and is able to support a maximum of 100 grams of bodily fluid specimen deposited within the collection cup **110,** and preferably without flexing and causing possible spillage of the deposited bodily fluid specimen. The hinged feature of the integral hinged lid **150** allows the integral hinged lid **150** to be pivoted into position over and above the collection cup **110** and snap-fastened with the collection cup **110** through the corresponding coupling element prepared on both the collection cup **110** and the integral hinged lid **150** mentioned earlier.

In an embodiment, the integral hinged lid **150** comprising a protruding rib **160** located in proximity to the hinged section thereof. The protruding rib **160** rests on the protruding rib support when the integral hinged lid is closed **150.** The protruding rib **160** is configured to interact with the test strip magazine **130** situated inside the collection cup **110** such that once the integral hinged lid **150** is closed the protruding rib **160** secures the test strip magazine **130** in place.

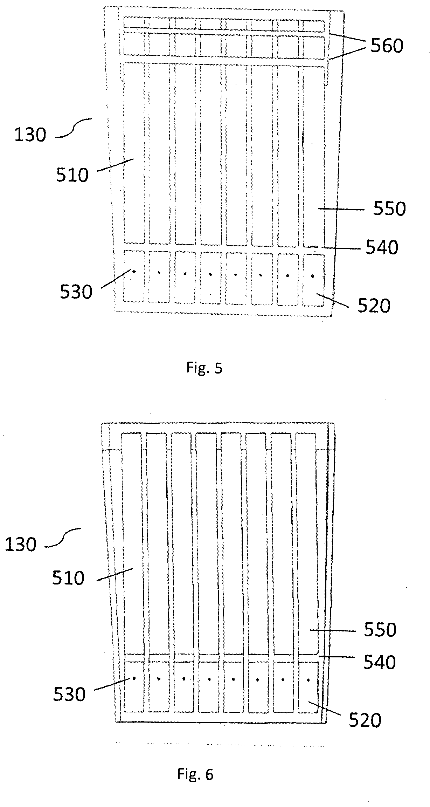

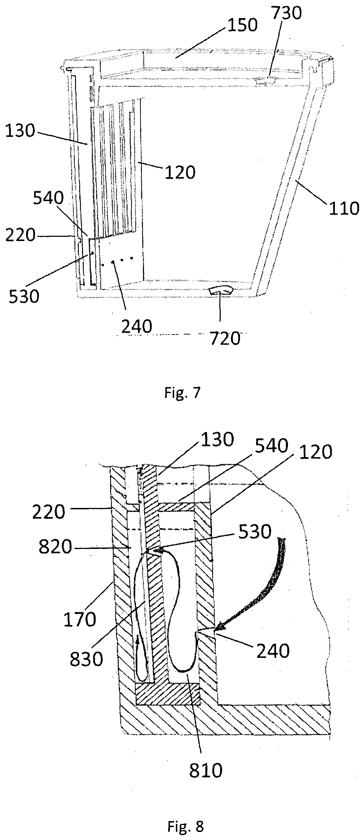

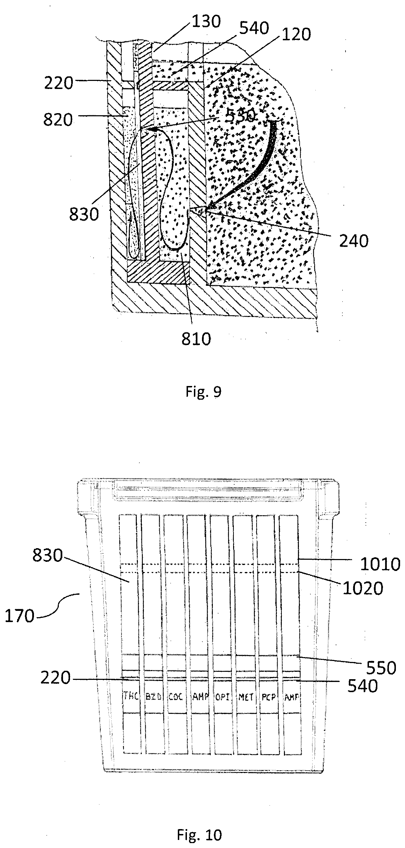

Fig. 2 illustrates a sectional perspective view of an embodiment of the device **100.** In this embodiment, the device **100** comprises a drainage port, the detail of which will be further described. Also illustrated in FIG. 2 is the test strip magazine **130** configured to fit tightly between the test strip viewing window **170** and the magazine supporting member **120.** The magazine supporting member **120** comprises a plurality of flow ports **240** disposed at the lower section of the magazine supporting member **120.** The numbers of the flow port **240** correspond to the number of recessed slots **510** prepared on the test strip magazine **130,** the detail of which will be further described. It is essential that each of the flow port **240** of the magazine supporting member **120** is located lower than the height of an outward flow port **530** prepared on the test strip magazine **130** and that it is also essential that the outward flow port **530,** must be located lower than the height of a damming rib **220** prepared on the wall of the collection cup **110** at which the assaying viewing window **170** is located. Further, the damming rib **220** is situated, preferably less than half the height of the assaying test strip viewing window **170** to match the height of the raised rib **540** (shown in Fig. 5). Once the test strip magazine **130** is inserted into the space between the assaying viewing window **170** and the magazine supporting member **120,** the damming rib **220,** the raised rib **540,** the test strip magazine **130,** and the magazine supporting member **120** together creates a mechanism to slow down the flow of the bodily fluid into the assaying test strip **830,** see FIGs. 8 and 9.

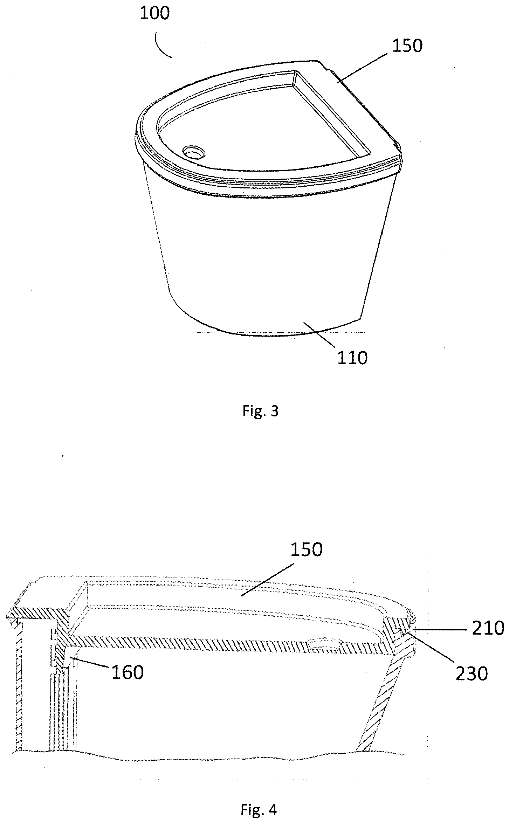

As mentioned earlier that both the collection cup **110** and the integral hinged lid **150** are prepared with corresponding coupling elements in order to provide a leak proof permanent seal to the device **100,** in an embodiment, the mentioned coupling elements are realized as, for example, the collection cup **110** comprises a groove **210** along an edge with a raised rib profile. The integral hinged lid **150** comprises a tongue **230** surrounding the edge of the integral hinged lid **150.** The tongue **230** is configured to be compatible with the groove **210** on the edge of the collection cup **110** such that coupling between them provides a tight leak proof permanent seal of the device **100** as illustrated in FIGs. 3 and 4.

After a bodily fluid specimen is deposited into the collection cup **110,** the integral hinged lid **150** is pivoted downward to engage with the collection cup **110** so as to close the device **100.** The coupling between the coupling element on the collection cup **110** and the integral hinged lid **150** provides a leak proof permanent seal to the device **100.** Once the device **100** is closed and as the coupling is intended to provide a permanent seal to the device **100** reopening of the device **100** without force is near impossible. If force is used to pry open the integral hinged lid **150** there is a good chance that there will be visual evidence (damages or deformation) to the device **100** suggesting that the sample may have been tampered with. Further, if the device **100** is submitted to stress or bending it will form a white section known as stress whitening. This reduces the risk of tampering or cross contamination of sample.

Further, as mentioned above, the integral hinged lid **150** also comprises the protruding rib **160** of which upon closing of the integral hinged lid **150,** the protruding rib **160** abut against the test strip magazine **130.** By such arrangement, the protruding rib **160** not only helps to secure the test strip magazine in place, but also provide an added seal to the device **100.** In more detail, the protruding rib **160** is located across the top of the test strip magazine **130.** The presence of the protruding rib **160** avoids the need for additional sealing membranes such as O-rings or additional seals to achieve a dynamic leak proof condition.

As illustrated in Fig. 5, there is shown front of the test strip magazine **130** detailing the recessed slots **510.** The front of the test strip magazine **130** is facing the main collection chamber **140.** The test strip magazine **130** is configured to be fitted between the magazine supporting member **120** and the assaying test strip viewing window **170.**

As mentioned above, the test strip magazine **130** comprises a plurality of recessed slots **510.** Each recessed slot **510** is configured to accommodate an assaying test strip **830** to be inserted to the said recessed slots **510.** The shape and configuration of the recessed slots **510** should correspond with the shape of the assaying test strip **830.** In an exemplary embodiment, the recessed slot **510** is rectangular in shape so as to accommodate a rectangular assaying test strip **830.**

Each recessed slot **510** comprises a damming device **520** each of which comprises an outward flow port **530.** Above the damming device **520** facing the main collection chamber **140** is a raised rib **540** which forms part of the damming device **520.** The damming device **520** is configured to serve the function of limiting the amount of bodily fluid specimen within and around the lower portion of the assaying test strips **830** (see Fig. 9). The raised rib **540** is preferably positioned below a conjugate zone **550** of the assaying test strip **830,** which is below the middle portion of the assaying test strip **830** to keep the level of bodily fluid below the conjugate zone **550.** The test strip conjugate zone **550** is where the reactive element takes place. Within the conjugate zone **550** there are a number of chemicals pre-coated with drug-protein conjugate for testing the presence of drugs in the bodily fluid. Thus failure to retain the flow level of the bodily fluid specimen well below the conjugate zone **550** may render the assaying test strip **830** ineffective or resulting in-inaccurate or inconclusive or false test results. The test strip magazine **130** comprises of a protruding rib support **560** near the top facing the main collection chamber **140** whereupon the hinged lid **150** is closed, the protruding rib **160** rests on the protruding rib support **560.**

Fig. 6 illustrates back of the test strip magazine **130.** The back of the test strip magazine **130** is facing the assaying test strip viewing window **170.** The recessed slots **510** with the damming device **520** are shown. The recessed slot **510** is dimensioned to correspond with the dimensions and configurations of the assaying test strip **830** such that each recessed slot **510** can accommodate an assaying test strip **830.** Preferably, each assaying test strip **830** is nested vertically into each individual recessed slot **510.** The assaying test strip **830** may be fitted by hand or by robotic machine in a clean room and zero humidity environment. The test strip magazine **130** may be preloaded with one or more assaying test strips **830.** In an embodiment, there are up to eight recessed slots **510** with eight assaying test strips **830** for testing wherein each assaying test strip is designated to be reactive to one specific substance or as an indicator of presence of such substance. This means that an array of different substances may be tested for, with only a single test using a single sufficient sample of specimen. The assaying test strip **830** is preferably made of an absorbent material and has a conjugate zone **550** that is positioned such that it allows the specimen to come into contact with or reacted with the assaying test strip **830.** In an embodiment, the conjugate zone **550** is positioned below the middle of the assaying test strip **830.**

Fig. 7 illustrates the sectional perspective view of an embodiment of the device **100** in a fully closed state. As shown, the magazine supporting member **120** comprises a plurality of flow port **240** wherein the said plurality of flow ports **240** disposed at the lower section of the magazine supporting member **120.** The numbers of the flow port **240** correspond to the number of recessed slots **510** prepared on the test strip magazine **130,** and each flow port is located a position corresponding to each recessed slot **510.** The flow port **240** is situated lower than the outward flow port **530** of the damming device **520.** This is to restrict the amount and pace of the fluid that is flowing towards the assaying test strip **830** as too much bodily fluid contacting the assaying test strip **380** at one time may result in false positive. The test strip magazine **130** has raised rib **540** in the front side facing the main collection chamber **140** and the raised rib **540** is prepared with the same height as the damming rib **220.** The raised rib **540** and the damming rib **220** together create a sealing effect so as to further reduce the bodily fluid pressure slowing down the flow of the bodily fluid. The raised rib **540** and damming rib **220** are dimensioned to correspond to one another such that they sufficiently and effectively encompass the assaying test strips **830** without having any adverse effects on or interfere with or restrict the capacity of the assaying test strip. For example, they must not squeeze the assaying test strips **830** too tightly such that it could restrict capillary fluid flow through the strips.

The main collection chamber **140** is configured to contain a volume of bodily fluid sample. In an embodiment, the collection chamber is capable of receiving up to 100 milliliters of bodily fluid specimen. In an embodiment, the bodily fluid specimen collection and assay device **100** comprising a drainage port **720** prepared at a main collection chamber **140** of the collection cup **110.** In an embodiment as shown in FIGs 2 and. 7, the drainage port **720** is prepared on the bottom region of the main collection chamber **140.** The drainage port **720** is provided as a practical means for discharging of the bodily fluid specimen from the main collection chamber **140** for disposal and or recycling purposes. In an embodiment, the drainage port **720** may be constructed from materials made to be easily penetrated with a suitable tool such that the fluid flowing may be discharged through the port and safely disposed of.

Further, in an embodiment, the device **100** comprising an extraction port **730,** disposed, preferably on the integral hinged lid **150.** As shown in FIGs 3 and 7, the integral hinged lid **150** comprises an extraction port **730** to permit extraction or retrieval of the sample contain inside the main collection chamber **140** without the need to unseal the hinged lid **150** from the collection cup **110.** As an example, the extraction port **730** may be made of self-resealed polymeric materials or membrane such that a sample of bodily fluid may be easily and safely extracted from the main collection chamber **140** using a suitable tool such as a hypodermic needle wherein the needle entry point is reseal upon withdrawal of the needle. With this characteristic, a secondary fluid specimen testing and analysis can be conducted without disengaging the sealed bodily fluid specimen collection and assay device **100.**

Fig. 8 illustrates the direction of the flow of the bodily fluid specimen from a main collection chamber **140** through the flow ports **240** into a pressure reduction chamber **810,** then through the outward flow ports **530** into an assaying test strip chamber **820** in a controlled hydraulic and preferred manner. The pressure reduction chamber **810** is formed as a result of engagement of the damming device **520,** the raised rib **540** and the magazine supporting member **120.** The assaying test strip chamber **820** is formed as a result of engagement of the assaying test strip viewing window **170,** the damming rib **220** and the test strip magazine **130.**

The damming device **520,** raised rib **540,** damming rib **220,** magazine supporting member **120,** flow port **240,** and outward flow port **530** are arranged so as to gradually reduce the fluid head pressure during the flow of the provided bodily fluid specimen from the main specimen collection chamber by restricting the flow of the bodily fluid specimen into a pressure reduction chamber **810** establishing a lesser fluid head pressure within the pressure reduction chamber **810.** The speed of fluid transfer flow from the main collection chamber **140** to the pressure reduction chamber **810** can be controlled by increasing or decreasing the flow port **240** diameter. This is to adjust the pressure so that the fluid does not contact the assaying test strip **830** too fast resulting in false result.

Fig. 9 illustrates a controlled flow of the provided bodily fluid specimen depicted by the flow directional arrow. Bodily fluid specimen is deposited into the main collection chamber **140** and the bodily fluid specimen moves into the pressure reduction chamber **810** via the flow ports **240** disposed on the magazine supporting member **120.** The bodily fluid specimen then flows from the pressure reduction chamber **810** into the assaying test strip chamber **820** very slowly via the outward flow ports **530** disposed on the test strip magazine **130.** The bodily fluid specimen seeps into the assaying test strip **830** and the test procedure commences. The damming rib **220** and the raised rib **540** are positioned adjacent to each other when the test strip magazine **130** is fitted into the magazine supporting member **120.** The damming rib **220** and the raised rib **540** provide pressure laterally and on either side of the assaying test strips **830.** Both the damming rib **220** and the raised rib **540** are located below the assaying test strip conjugate zone **550.** The damming rib **220** and the raised rib **540** ensure that the bodily fluid specimen entering the assaying test strip chamber **820** only reaches the preferred operable fluid level and in relation to the positioning of the assaying test strip conjugate zone **550.**

As mentioned above, the bodily fluid collection cup **110** comprises a bottom with a surrounding wall to define a main collection chamber **140.** The said wall defines an assaying test strip viewing window **170** to facilitate viewing of the test results. Fig. 10 illustrates the bodily fluid collection cup **110** with the assaying test strip viewing window **170** having assaying test strips **830** positioned in place and within the test strip magazine **130.** The assaying test strip viewing window **170** is transparent so that test result can be viewed through the assaying test strip viewing window **170** without having to open the bodily fluid specimen collection and assay device **100.** The assaying test strip viewing window **170** and the assaying test strips **830** are clearly visible when the device is placed on its base on a flat surface such as a table.

As mentioned, the assaying test strip viewing window **170** comprises a damming rib **220.** The preferred position of the damming rib **220** and the raised rib **540** are shown in relation to the assaying test strip conjugate zone **550** and the assaying test strip control region **1010** and test region **1020,** which indicates the test results. The bodily fluid specimen collection and assay device **100** is made by injection molding where the bodily fluid collection cup **110** and the integral hinged lid **150** are produced as one piece. The bodily fluid specimen collection and assay device **100** is made of polymer. The bodily fluid specimen collection and assay device **100** is produced using a common injection molding grade of plastic resin, for example Random Copolymer Polypropylene. Thus the material is quite soft. If a hand tool is used to open the integral hinged lid **150** there will be visual evidence. The plastic whitens if submitted to stress or bending. The whitening of the plastic is a good indicator that the bodily fluid specimen collection and assay device **100** has been tampered with.

The bodily fluid specimen collection and assay device **100** comprises at least an area for identification labeling. The bodily fluid specimen collection and assay device 100 can be labeled using a writing instrument or by etching. The etching is useful for labeling the subject's identification and the name of specific assays, at the same time eliminating the need for expensive adhesive labels and other decorative components. The bodily fluid specimen collection and assay device **100** comprises a shape of a standard toilet bowl and lid.

The present invention is more cost- effective and less complicated to manufacture when compared to other known and commercially available bodily fluid specimen collection devices. The bodily fluid specimen collection and assay device 100 is in one piece, convenient and easy to use.

## Claims

1. A bodily fluid specimen collection and assay device comprising:
- a bodily fluid collection cup comprising a bottom and a surrounding wall defining a main collection chamber and an assaying test strip viewing window define on the said wall of the bodily fluid collection cup comprising a damming rib;
- an integral hinged lid integrally hinged to the said bodily fluid collection cup;
- a magazine supporting member disposed within the main collection chamber, the said magazine supporting member comprising a plurality of flow ports disposed at the lower section of the magazine supporting member
- a test strip magazine comprising:
a plurality of recessed slots each of which is configured to accommodate an assaying test strip;
a damming device disposed on each recessed slot;
an outward flow port disposed on each damming device; disposed at a position higher than the position of the flow ports on the magazine supporting member; and
a raised rib positioned to match the height of the damming rib;
wherein the damming rib, the raised rib, the test strip magazine and the magazine supporting member engaged to create a differential pressure chamber.

2. The bodily fluid specimen collection and assay device of claim 1, wherein the bodily fluid collection cup and the integral hinged lid are prepared with corresponding coupling element configured to provide a leak proof permanent seal to the bodily fluid specimen collection and assay device.

3. The bodily fluid specimen collection and assay device of claim 2, wherein the bodily fluid specimen collection and assay device further comprising an extraction port prepared on the integral hinged lid.

4. The bodily fluid specimen collection and assay device of claim 2 wherein the bodily fluid specimen collection and assay device further comprising a drainage port prepared on the bottom of the main collection chamber of the bodily fluid collection cup.

5. The bodily fluid specimen collection and assay device of claim 3 wherein the bodily fluid specimen collection and assay device further comprising a drainage port prepared on the bottom of the main collection chamber of the bodily fluid collection cup.

6. The bodily fluid specimen collection and assay device of claim 2 or 5, wherein the integral hinged lid further comprising a protruding rib support along top of the test strip magazine.

7. The bodily fluid specimen collection and assay device of claim 6, wherein the integral hinged lid further comprising a protruding rib configured to rest on the protruding rib support on the -integral hinged lid as the integral hinged lid is coupled to the bodily fluid collection cup.

8. The bodily fluid specimen collection and assay device of any one of claims 1-7, wherein the recessed slot is of a shape corresponding to a shape of a test strip.

9. The bodily fluid specimen collection and assay device of claim 1, wherein the test strip magazine comprising up to eight recessed slots.

10. The bodily fluid specimen collection and assay device of claim 1 comprising at least an area for identification labeling.

11. The bodily fluid specimen collection and assay device of claim 1 comprising an extraction port comprising a self-resealed polymeric material or membrane.

## Patentansprüche

1. Vorrichtung zur Körperflüssigkeits-Probenahme und -Assay, aufweisend:
- einen Körperflüssigkeits-Probenahmebecher mit einem Boden und einer Mantelwand, womit eine Probenahme-Hauptkammer definiert ist, und ein Prüfteststreifen-Sichtfenster, das auf der Wand des Körperflüssigkeits-Probenahmebechers mit einer Stauleiste definiert ist,
- einen integralen Klappdeckel, der an dem Körperflüssigkeits-Probenahmebecher einstückig angelenkt ist,
- ein Magazin-Träger-Element, das in der Probenahme-Hauptkammer angeordnet ist, wobei das Magazin-Träger-Element eine Vielzahl von an dem unteren Abschnitt des Magazin-Träger-Elements angeordneten Durchflussöffnungen aufweist,
- ein Magazin für Teststreifen mit
einer Vielzahl von vertieften Schlitzen, von denen jeder zur Aufnahme eines Prüfteststreifens eingerichtet ist,
einer Staueinrichtung, die auf jedem vertieften Schlitz angeordnet ist,
einer auswärts gerichteten Durchflussöffnung, die auf jeder Staueinrichtung angeordnet ist, in einer Position höher als die Position der Durchflussöffnungen auf dem Magazin-Träger-Element, und
einer erhöhten Leiste, die der Höhe der Stauleiste entsprechend positioniert ist,
wobei die Stauleiste, die erhöhte Leiste, das Magazin für Teststreifen und das Magazin-Träger-Element zum Ausbilden einer Differenzialdruckkammer zusammentreten.

2. Vorrichtung zur Körperflüssigkeits-Probenahme und -Assay nach Anspruch 1, wobei der Körperflüssigkeits-Probenahmebecher und der integrale Klappdeckel mit einem korrespondierenden Kopplungselement gebildet sind, das zum Herstellen einer leckdichten dauerhaften Abdichtung für die Vorrichtung zur Körperflüssigkeits-Probenahme und -Assay ausgebildet ist.

3. Vorrichtung zur Körperflüssigkeits-Probenahme und -Assay nach Anspruch 2, welche ferner eine auf dem integralen Klappdeckel gebildete Extraktionsöffnung aufweist.

4. Vorrichtung zur Körperflüssigkeits-Probenahme und -Assay nach Anspruch 2, welche ferner eine Abflussöffnung aufweist, die auf dem Boden der Probenahme-Hauptkammer des Körperflüssigkeits-Probenahmebechers gebildet ist.

5. Vorrichtung zur Körperflüssigkeits-Probenahme und -Assay nach Anspruch 3, welche ferner eine Abflussöffnung aufweist, die auf dem Boden der Probenahme-Hauptkammer des Körperflüssigkeits-Probenahmebechers gebildet ist.

6. Vorrichtung zur Körperflüssigkeits-Probenahme und -Assay nach Anspruch 2 oder 5, wobei der integrale Klappdeckel ferner ein Vorsprungsleistenlager entlang des Oberteils des Magazins für Teststreifen aufweist.

7. Vorrichtung zur Körperflüssigkeits-Probenahme und -Assay nach Anspruch 6, wobei der integrale Klappdeckel ferner eine vorspringende Leiste aufweist, die dazu eingerichtet ist, auf dem Vorsprungsleistenlager auf dem integralen Klappdeckel anzuliegen, wenn der der integrale Klappdeckel an dem Körperflüssigkeits-Probenahmebecher angekoppelt ist.

8. Vorrichtung zur Körperflüssigkeits-Probenahme und -Assay nach einem der Ansprüche 1 bis 7, wobei der vertiefte Schlitz eine Formgebung hat, die der Formgebung eines Teststreifens entspricht.

9. Vorrichtung zur Körperflüssigkeits-Probenahme und -Assay nach Anspruch 1, wobei das Magazin für Teststreifen bis zu acht vertiefte Schlitze aufweist.

10. Vorrichtung zur Körperflüssigkeits-Probenahme und -Assay nach Anspruch 1, aufweisend zumindest einem Bereich für eine Identifikations-Beschriftung.

11. Vorrichtung zur Körperflüssigkeits-Probenahme und -Assay nach Anspruch 1, aufweisend eine Extraktionsöffnung mit einem/einer selbsttätig wiederverschlossenen Polymermaterial oder Membran.

## Revendications

1. - Dispositif de collecte et d'analyse d'échantillon de fluide corporel comprenant :
- une coupelle de collecte de fluide corporel comprenant un fond et une paroi périphérique définissant une chambre de collecte principale et une fenêtre de visualisation de bâtonnets diagnostiques d'analyse définie sur ladite paroi de la coupelle de collecte de fluide corporel comprenant une nervure de retenue ;
- un couvercle articulé intégré, articulé de manière intégrée à ladite coupelle de collecte de fluide corporel ;
- un élément de support de magasin disposé à l'intérieur de la chambre de collecte principale, ledit élément de support de magasin comprenant une pluralité d'orifices d'écoulement disposés au niveau de la section inférieure de l'élément de support de magasin ;
- un magasin pour bâtonnets diagnostiques comprenant :
une pluralité de fentes renfoncées, chacune configurée pour recevoir un bâtonnet diagnostique d'analyse ;
un dispositif de retenue disposé sur chaque fente renfoncée ;
un orifice d'écoulement vers l'extérieur disposé sur chaque dispositif de retenue ; disposé dans une position plus haute que la position des orifices d'écoulement sur l'élément de support de magasin ; et
une nervure surélevée positionnée pour correspondre à la hauteur de la nervure de retenue ;
la nervure de retenue, la nervure surélevée, le magasin pour bâtonnets diagnostiques et l'élément de support de magasin étant engagés pour créer une chambre à pression différentielle.

2. - Dispositif de collecte et d'analyse d'échantillon de fluide corporel selon la revendication 1, dans lequel la coupelle de collecte de fluide corporel et le couvercle articulé intégré sont préparés avec un élément de couplage correspondant configuré pour fournir au dispositif de collecte et d'analyse d'échantillon de fluide corporel un scellement permanent étanche aux fuites.

3. - Dispositif de collecte et d'analyse d'échantillon de fluide corporel selon la revendication 2, le dispositif de collecte et d'analyse d'échantillon de fluide corporel comprenant en outre un orifice d'extraction préparé sur le couvercle articulé intégré.

4. - Dispositif de collecte et d'analyse d'échantillon de fluide corporel selon la revendication 2, le dispositif de collecte et d'analyse d'échantillon de fluide corporel comprenant en outre un orifice de drainage préparé sur le fond de la chambre de collecte principale de la coupelle de collecte de fluide corporel.

5. - Dispositif de collecte et d'analyse d'échantillon de fluide corporel selon la revendication 3, le dispositif de collecte et d'analyse d'échantillon de fluide corporel comprenant en outre un orifice de drainage préparé sur le fond de la chambre de collecte principale de la coupelle de collecte de fluide corporel.

6. - Dispositif de collecte et d'analyse d'échantillon de fluide corporel selon la revendication 2 ou 5, dans lequel le couvercle articulé intégré comprend en outre un support de nervure en saillie le long de la partie supérieure du magasin pour bâtonnets diagnostiques.

7. - Dispositif de collecte et d'analyse d'échantillon de fluide corporel selon la revendication 6, dans lequel le couvercle articulé intégré comprend en outre une nervure en saillie configurée pour reposer sur le support de nervure en saillie sur le couvercle articulé intégré lorsque le couvercle articulé intégré est couplé à la coupelle de collecte de fluide corporel.

8. - Dispositif de collecte et d'analyse d'échantillon de fluide corporel selon l'une quelconque des revendications 1 à 7, dans lequel la fente renfoncée est d'une forme correspondant à une forme d'un bâtonnet diagnostique.

9. - Dispositif de collecte et d'analyse d'échantillon de fluide corporel selon la revendication 1, dans lequel le magasin pour bâtonnets diagnostiques comprend jusqu'à huit fentes renfoncées.

10. - Dispositif de collecte et d'analyse d'échantillon de fluide corporel selon la revendication 1, comprenant au moins une zone pour étiquetage d'identification.

11. - Dispositif de collecte et d'analyse d'échantillon de fluide corporel selon la revendication 1, comprenant un orifice d'extraction comprenant une membrane ou matériau polymère auto-refermable.
